Europäisches Patentamt

European Patent Office

Office européen des brevets

(19)

(11) Publication number: **0 223 370 B1**

## EUROPEAN PATENT SPECIFICATION

(12)

(45) Date of publication of patent specification: 02.05.91   (51) Int. Cl.⁵: **A61F 2/08**, A61B 17/56

(21) Application number: 86307590.9

(22) Date of filing: 02.10.86

(54) Prosthetic ligament.

(30) Priority: 17.10.85 GB 8525565

(43) Date of publication of application:
27.05.87 Bulletin 87/22

(45) Publication of the grant of the patent:
02.05.91 Bulletin 91/18

(84) Designated Contracting States:
AT BE CH DE ES FR IT LI LU NL SE

(56) References cited:
EP-A- 0 051 954      EP-A- 0 126 520
EP-A- 0 153 831      WO-A-84/04669
GB-A- 2 097 260      US-A- 3 973 277

(73) Proprietor: Seedhom, Bahaa Botros
75 Holt Park Crescent
Leeds 16 West Yorkshire(GB)

Proprietor: Fujikawa, Kyosuke
Department of Orthopaedic Surgery Keio
School of Medicine
Tokyo(JP)

(72) Inventor: Seedhom, Bahaa Botros
75 Holt Park Crescent
Leeds 16 West Yorkshire(GB)
Inventor: Fujikawa, Kyosuke
Department of Orthopaedic Surgery Keio
School of Medicine
Tokyo(JP)

(74) Representative: Orr, William McLean et al
URQUHART-DYKES & LORD 5th Floor, Tower
House Merrion Way
Leeds West Yorkshire, LS2 8PA(GB)

## Description

This invention relates to a prosthetic ligament for implantation between at least two bones.

It is known from EP-A-0126520 to provide a prosthetic ligament for implantation between at least two bones and which comprises a flexible elongate body made of foraminous material, and having pulling means attached to one end of the body for threading and for pulling the ligament through channels formed in the bones.

It is also known from EP-A-0051954 to provide a detachable sheath of flexible material which extends substantially along the whole of the length of the body of a prosthetic ligament, such sheath being attached initially to the ligament for joint movement with the ligament throught the channels in the bones and thereafter being detachable from the ligament to allow the sheath to be removed immediately after implantation of the ligament.

However, in the prosthetic ligament known from EP-0051954, there is no pulling means provided to assist the threading and pulling of the ligament through channels formed in bones of a joint, but in addition the sheath is positioned around the ligament in such a way that it can only be removed by being cut longitudinally, and therefore it is an awkward task to achieve complete removal of the sheath after the ligament and sheath have been introduced through the channels in the bones.

The present invention has therefore been developed with a view to providing a prosthetic ligament structure which can be readily implanted, and which has a sheath which protects the ligament during implantation, but which has means which provides for improved and easier removal of the sheath after implantation.

According to the invention there is provided a prosthetic ligament which comprises a flexible elongate body adapted for implantation between at least two bones, pulling means attached to one end of the body for threading and for pulling the ligament through channels formed in the bones, characterised by a detachable sheath of flexible material extending substantially along the whole of the length of the body of the ligament and attached at one end to the pulling means for joint movement with the ligament body through the channels in the bones, said sheath being detachable from the ligament body to allow the sheath to be removed after implantation of the ligament body and sheath.

Thus, a prosthetic ligament according to the invention can be readily introduced through channels formed in the bones by use of the pulling means, and during this implantation of the ligament, the sheath protects the ligament body from contact with the inner wall of the channels, thereby preventing the ligament (which is of woven material) from risk of snagging with the rough inner surface of the bone channels, which would otherwise cause damage to the ligament and also impede its passage through the channels. In addition, by virtue of the detachable connection between the end of the sheath and the pulling means, the sheath can readily be removed from its protective position over the ligament after implantation, by exerting a pulling action on the end of the sheath remote from the pulling means.

Preferably, the pulling means comprises a cord, and which may be attached to said one end of the sheath by means of a knot.

Conveniently, said one end of the sheath extends beyond the point of attachment of the cord to the ligament body, and the cord is knotted in a further knot within the sheath, the sheath being slidably removable from the ligament body upon cutting through said one end of the sheath and through the cord between the first mentioned knot and the further knot.

To provide a gripping portion which can be readily manipulated, to permit removal of the sheath, preferably the opposite end of the sheath extends beyond an end of the ligament body remote from the pulling means.

The sheath may take the form of a tube, or consists of a plurality of separate pieces of material.

One embodiment of prosthetic ligament according to the invention will now be described in detail, by way of example only, with reference to the accompanying drawings, in which:

Figure 1 shows the closed end of one embodiment of ligament with a sheath secured thereto;

Figure 2 shows the open end of the ligament surrounded by the sheath;

Figure 3 shows the sheathed ligament being threaded through the channels in the tibia and femur;

Figure 4 shows the sheath and cord being cut;

Figure 5 shows the ligament being pulled taut and the sheath being withdrawn from the ligament;

Figure 6 shows a bone plug being placed within the pouch of the ligament;

Figure 7 shows the ligament being pulled to secure the femoral bone plug in place in the femur; and

Figure 8 shows the implanted ligament with the sheath completely withdrawn;

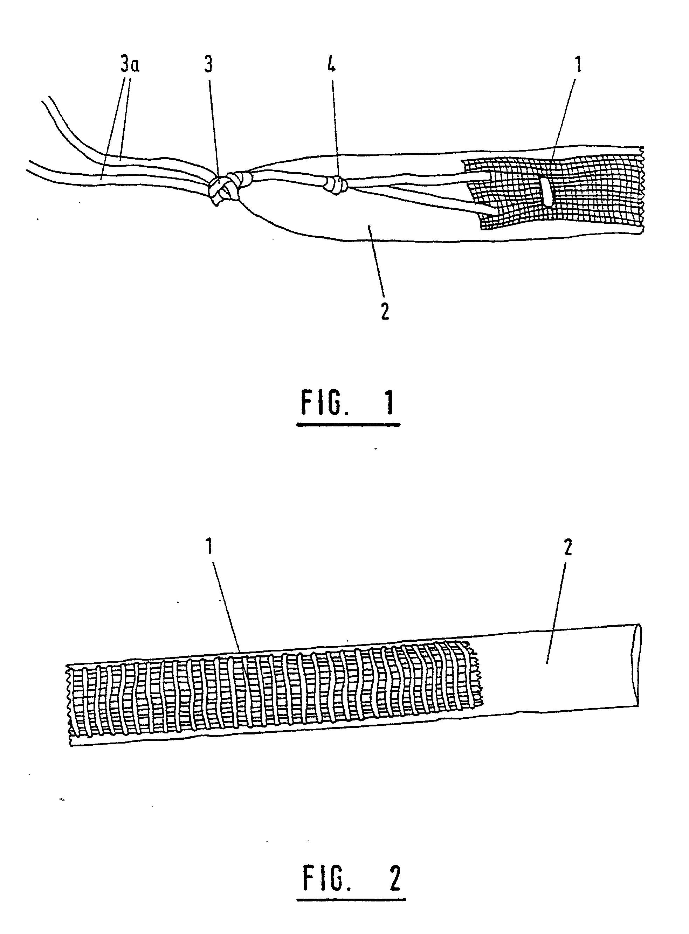

Referring to Figure 1, a ligament 1 of the present invention takes the form of an elongate strip of open weave polyester yarn which is formed into a tube. One example of a possible construction of the ligament 1 is shown in more detail in published European specification No 0153831A which takes the form of a perforated tube open at one end, and having a pouch at its opposite end.

The ligament 1 is provided with a sheath 2 in the form of a tube made from polyethylene sheet. The sheath 2 completely surrounds the ligament 1 along all of its length, and is secured at the closed end of the ligament by means of a knot 3 in the cord 3a which is attached to the closed end of the ligament. The knot 3 is knotted around the outside of the sheath 2 to secure it in place and prevent sliding of the sheath 2 with respect to the ligament 1. The cord 3a is also knotted within the sheath in a knot 4.

Referring to Figure 2, the opposite end of the sheath is open and extends a little way beyond the open end of the ligament.

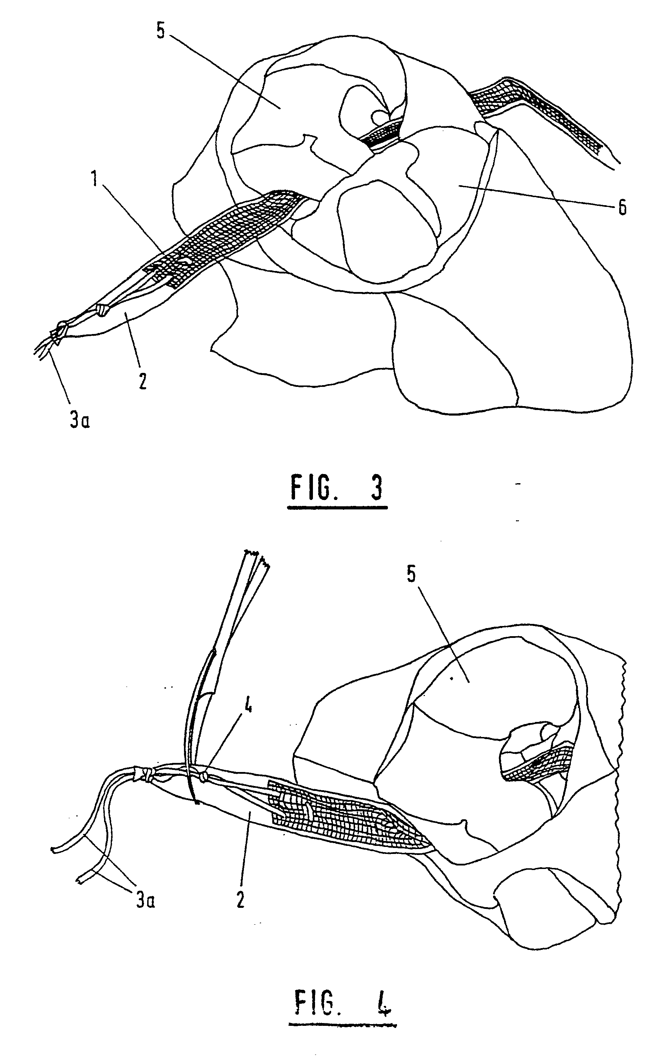

Referring to Figure 3, after the surgical operation of removing bone plugs from the femur 5 and the tibia 6 and drilling holes extending from the base of each of the sockets to the opposite side of the respective bone, the sheathed ligament is threaded through the channels, first through the tibia and then through the femur by means of pulling on the cord 3a. The sheath 2 serves to protect the ligament 1 as it is pulled through the channels in the bones, and also allows easy passage of the ligament through the bones without snagging of the ligament material.

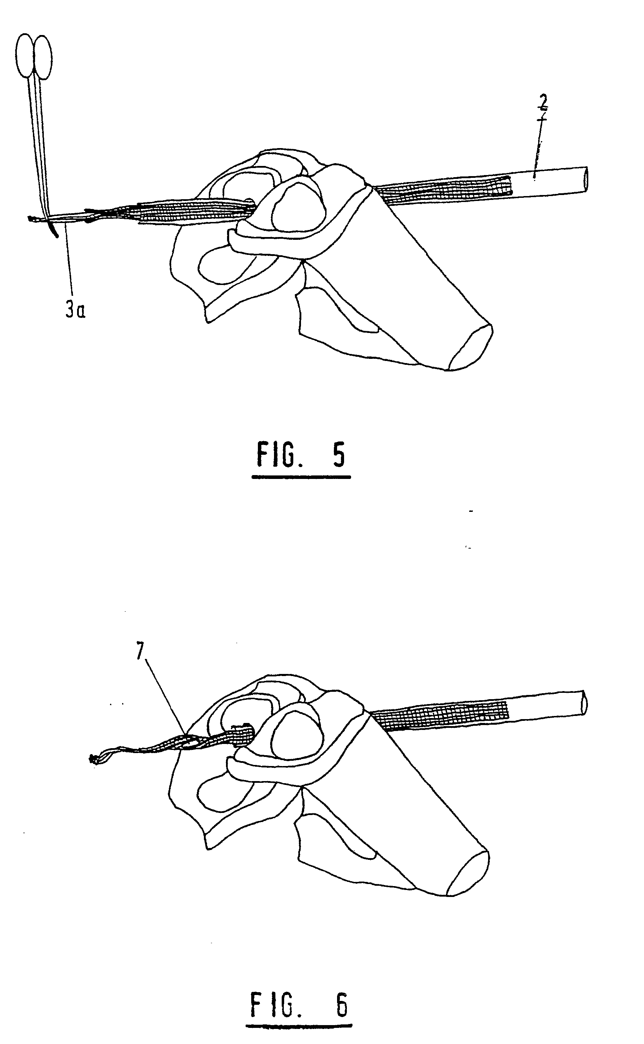

Referring now to Figure 4, once the ligament has been threaded through the bones, the sheath 2 and the cord 3a are cut just above the knot 4. This allows the sheath to side freely with respect to the ligament.

Referring now to Figure 5, the ligament is pulled taut by holding the cord 3a and at the same time pulling on the sheath 2 at the open end of the ligament.

Referring to Figure 6, the sheath is partly withdrawn from the ligament, exposing the pouch, and the femoral bone plug is placed into the pouch 7 at the closed end of the ligament.

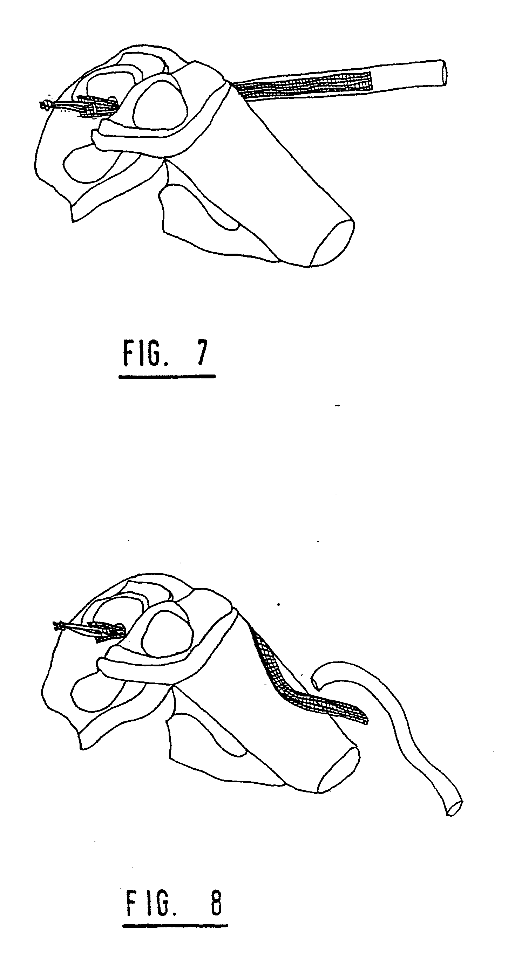

Referring to Figure 7, the ligament is then pulled at the open end causing the bone plug in the pouch to be pulled into place within the pouch in the femoral socket.

Figure 8 shows the ligament having had the sheath completely withdrawn from it and the femoral bone plug in place.

## Claims

1. A prosthetic ligament which comprises a flexible elongate body (1) adapted for implantation between at least two bones, (5,6) pulling means (3a) attached to one end of the body (1) for threading and for pulling the ligament through channels formed in the bones, characterised by a detachable sheath (2) of flexible material extending substantially along the whole of the length of the body (1) of the ligament and attached at one end to the pulling means (3a) for joint movement with the ligament body (1) through the channels in the bones, said sheath (2) being detachable from the ligament body (1) to allow the sheath to be removed after implantation of the ligament body (1) and sheath (2).

2. A prosthetic ligament according to Claim 1, characterised in that the pulling means comprises a cord (3a).

3. A prosthetic ligament according to Claim 2, characterised in that the cord (3a) is attached to said one end of the sheath (2) by means of a knot (3).

4. A prosthetic ligament according to Claim 3, characterised in that said one end of the sheath (2) extends beyond the point of attachment of the cord (3a) to the ligament body (1), and the cord is knotted in a further knot (4) within the sheath, the sheath being slidably removable from the ligament body upon cutting through said one end of the sheath and through the cord between the first mentioned knot (3) and the further knot (4).

5. A prosthetic ligament according to any one of Claims 1 to 4, characterised in that the opposite end of the sheath extends beyond an end of the ligament body (1) remote from the pulling means (3a).

6. A prosthetic ligament according to any one of Claims 1 to 5, characterised in that the sheath (2) is in the form of a tube, or consists of a plurality of separate pieces of material.

## Revendications

1. Prothèse de ligament comprenant un corps allongé flexible (1) adapté pour être implanté entre au moins deux os (5,6), des moyens de traction (3a) fixés à une extrémité du corps (1) pour enfiler et pour tirer le ligament à travers des conduits formés dans les os, caractérisée en ce qu'une gaine détachable (2) en matière flexible s'étend sensiblement sur toute la longueur du corps (1) du ligament et est fixée par une extrémité aux moyens de traction (3a) pour être déplacée solidairement avec le corps de ligament (1) à travers les conduits des os, ladite gaine (2) étant détachable du corps de ligament (1) pour permettre à la gaine d'être enlevée après l'implantation du corps de ligament (1) et de la gaine (2).

2. Prothèse de ligament selon la revendication 1, caractérisée en ce que les moyens de traction comportent un cordon (3a).

3. Prothèse de ligament selon la revendication 2, caractérisée en ce que le cordon (3a) est fixé à ladite extrémité de la gaine (2) au moyen d'un noeud (3).

4. Prothèse de ligament selon la revendication 3, caractérisée en ce que ladite extrémité de la gaine (2) s'étend au-delà du point de fixation du cordon (3a) sur le corps de ligament (1), et en ce que le cordon est noué par un autre noeud (4) dans la gaine, la gaine pouvant être désolidarisée du corps de ligament en la faisant glisser après avoir coupé ladite extrémité de la gaine et le cordon entre le noeud (3) mentionné en premier et l'autre noeud (4).

5. Prothèse de ligament selon l'une quelconque des revendications 1 à 4, caractérisée en ce que l'extrémité opposée de la gaine s'étend au-delà d'une extrémité du corps de ligament (1), éloignée des moyens de traction (3a).

6. Prothèse de ligament selon l'une quelconque des revendications 1 à 5, caractérisée en ce que la gaine (2) a la forme d'un tube, ou est constituée de plusieurs morceaux de matière distincts.

**Ansprüche**

1. Bandprothese, die einen flexiblen langgestreckten Hauptteil (1), der für eine Implantation zwischen wenigstens zwei Knochen (5, 6) ausgebildet ist, und eine Zugeinrichtung (3a) umfaßt, die an dem einen Ende des Hauptteils (1) befestigt ist, um das Band durch Kanäle zu fädeln und zu ziehen, die in den Knochen ausgebildet sind, **gekennzeichnet** durch eine lösbare Hülle (2) aus flexiblem Material, die sich im wesentlichen längs der gesamten Länge des Hauptteils (1) des Bandes erstreckt und an einem Ende mit der Zugeinrichtung (3a) für eine gemeinsame Bewegung mit dem Band-Hauptteil (1) durch die Kanäle in den Knochen befestigt ist, wobei die Hülle (2) von dem Band-Hauptteil (1) lösbar ist, um es zu ermöglichen, daß die Hülle nach der Implantation des Band-Hauptteils (1) und der Hülle (2) entfernt wird.

2. Bandprothese nach Anspruch 1, dadurch **gekennzeichnet**, daß die Zugeinrichtung eine Schnur (3a) umfaßt.

3. Bandprothese nach Anspruch 2, dadurch **gekennzeichnet**, daß die Schnur (3a) an dem einen Ende der Hülle (2) vermittels eines Knotens (3) befestigt ist.

4. Bandprothese nach Anspruch 3, dadurch **gekennzeichnet**, daß das eine Ende der Hülle (2) sich über den Befestigungspunkt der Schnur (3a) am Band-Hauptteil (1) hinauserstreckt und daß die Schnur innerhalb der Hülle zu einem weiteren Knoten (4) geknotet ist, wobei die Hülle nach einem Durchschneiden des einen Endes der Hülle und der Schnur zwischen dem zuerst genannten Knoten (3) und dem weiteren Knoten (4) vom Band-Hauptteil entfernbar ist.

5. Bandprothese nach einem der Ansprüche 1 bis 4, dadurch **gekennzeichnet**, daß sich das gegenüberliegende Ende der Hülle über ein Ende des Band-Hauptteils (1) hinauserstreckt, das von der Zugeinrichtung (3a) entfernt liegt.

6. Bandprothese nach einem der Ansprüche 1 bis 5, dadurch **gekennzeichnet,** daß die Hülle (2) die Form eines Schlauches besitzt oder aus einer Vielzahl von getrennten Materialteilen besteht.

## FIG. 1

## FIG. 2

EP 0 223 370 B1

FIG. 3

FIG. 4

**FIG. 5**

**FIG. 6**

FIG. 7

FIG. 8